# EUROPEAN PATENT APPLICATION

(11) **EP 1 518 560 A1**
(43) Date of publication of application: **30.03.2005**
(21) Application number: 03445106.2
(22) Date of filing: 26.09.2003
(51) Int. Cl.: A61K 49/00, A61K 33/00, A61K 33/16, A61K 31/01, A61K 31/045

(54) **Use of a gas for the manufacture of a diagnosing agent for diagnosing coronary perfusion**

(71) Applicant: AGA AB, 181 81 Lidingö (SE)
(72) Inventor: Piros, David, 111 38 Stockholm (SE)
(74) Representative: Larsson, Kjell

(57) **Abstract**

Use of a gas, or gas precursor, which is of such a nature that it is detectable via the expired breath from a mammal, for the manufacture of a diagnostically acceptable, injectable diagnosing agent for diagnosing coronary perfusion in said mammal, including man. A method and a diagnosing agent for such diagnosing.

## Description

### Technical field

The present invention is within the field of diagnosing coronary perfusion in a mammal, including man. More specifically, it relates to the use of a gas, or gas precursor, which is detectable via the expired breath from said mammal.

### Background art

Approximately one third of all deaths in the affluent society of the western world results from coronary heart diseases, and almost all elderly persons have at least some impairment of the coronary artery circulation. Therefore, there is a need for better diagnostic methods in this area.

The working myocardium is supplied with oxygenated blood from the right half of the heart by two blood vessels. The vessels divide into several smaller branches, from which the blood perfuses the entire heart, before entering into the left half of the heart. Since there are few overlaps between these arteries, problems with supply of blood to the heart will occur if any of these arteries are obstructed. If the supply of oxygenated blood to the myocardial tissues is obstructed, the muscle fibers will start to die.

Existing methods for diagnosing coronary perfusion comprise myocardial scintigraphy. Radioactive isotopes are thus administered intravenously to a patient, causing the radioactive nucleotides to be absorbed in perfused organs and tissues. Thus, it is possible to estimate the perfusion through various parts of the heart with a scintigraph. However, this method is expensive, time consuming and requires experienced personnel. Moreover, the method can not be immediately reproduced, since there is a risk of accumulation of radioactive nucleotides. An overall picture of the coronary perfusion of the heart cannot either be obtained.

Another method for diagnosing coronary perfusion is coronary angiography. In coronary angiography, a catheter is inserted through the femoral artery, advanced through the aorta and into a specific coronary artery. By injection of X-ray contrast medium through the catheter, occlusions and obstructions of coronary arteries can be seen by continuous X-ray monitoring. This method also demands experienced personnel for interpretation. Exposing the patient to X-rays is not good for the patient. Another drawback is that small obstructions are not seen. A functional estimate of the coronary perfusion is furthermore not obtained.

US 4,610,258 discloses a method for determining tissue blood flow, more particularly the cerebral blood flow, by using a freely diffusible inert gas, such as xenon, as a contrasting agent and computer tomography (CT) for detecting the gas, by calculating a flow rate constant and a partition coefficient. However, using CT is an expensive method, which requires highly trained personnel.

Thus, common for all known methods is that they are complicated and require trained personnel for the performances thereof. Moreover, the known methods are usually expensive, may harm the patient and do not give an overall picture of the coronary perfusion of the heart.

### Summary of the invention

The present invention eliminates problems referred to above by providing a completely new and simple technique for diagnosing coronary perfusion.

When a gas is administered to a coronary artery of a mammal, including man, said gas will be brought to the lungs and thus be present for detection in the expired breath from said mammal. The present invention is based on the finding that the pattern over time of the concentration of the gas in the expired breath is related to the coronary perfusion of the mammal in question. Accordingly, satisfactory perfusion will be indicated by a sharp concentration peak in expired breath, whereas impaired perfusion will be indicated by deflections of the curve.

More specifically, according to a first aspect of the present invention, there is provided use of a gas, or gas precursor, which is of such a nature that it is detectable via the expired breath from a mammal, for the manufacture of a diagnostically acceptable, injectable diagnosing agent for diagnosing coronary perfusion in said mammal, including man.

A second aspect of the invention is represented by a method for diagnosing coronary perfusion in a mammal, including man, which comprises administration to a coronary artery of said mammal a gas or gas precursor, which is of such a nature that it is detectable via the expired breath from said mammal, and monitoring the expired breath from said mammal by means of a gas detector to detect the gas therein.

Still another aspect of the invention, or expressed in another way, there is also provided a diagnosing agent for diagnosing coronary perfusion in a mammal, including man, which comprises a diagnostically acceptable gas or gas precursor, which is of such a nature that it is detectable via the expired breath from said mammal.

Measurement of gas in the exhaled air is a suitable way to detect dissolved gases injected into the coronary circulation since the gas only needs to travel a very short distance from the working myocardium before the lung is reached. Short-term changes in the pattern of gas release from the lung can therefore be explained by changes in coronary perfusion.

When said gas is used in this way as a diagnosing agent, already small amounts thereof can be detected rapidly. In other words, the present invention enables the use of a new diagnosing technique which is much faster than known techniques. An overall picture of the coronary perfusion can thus be obtained. Furthermore, no expensive equipment is needed.

In addition, the new technique is simple and less expensive, as there will for instance not be needed any visual determinations by highly competent or trained personnel. No blood sampling or any other more or less complicated blood or flow measurements are needed. The gases are in addition not harmful to the patient, and the patient will not be subjected to radioactivity or contrast medium as in prior art methods.

Moreover, detection of e.g. exhaled nitrous oxide is very accurate and can be made with simple and even existing nitrous oxide monitors. For instance, such monitors are available in anesthesia machines, which monitors may be used as such or easily converted into more accurate monitors. The monitoring can also be automated.

The invention is of great importance in connection with care of very ill patents in cardiology (heart diseases).

Other objects of or advantages with the invention should be apparent to a person skilled in the art after having read the description below.

### Detailed description of preferred embodiments of the invention

In one embodiment of the present invention, said diagnosing is for the detection of disturbances of the coronary perfusion.

In another embodiment of the present invention, said diagnosing is for the detection of stunned myocardium, whereby the mobility of the myocardium is impaired.

Said diagnosing can also be for the detection of myocardial infarction. When the supply of oxygen via the blood to the myocardium is not sufficient, the muscle fibers in the affected area will start to die. If the area is large, the condition is lethal.

In yet another embodiment of the present invention, said diagnosing is for the detection of angina pectoris, which is a preliminary stage of myocardial infarction, and often the reason to perform a study of the coronary perfusion. Angina pectoris is caused by lack of oxygenated blood to the myocardium. The pain caused by angina pectoris mostly occurs whenever the load on the heart becomes greater than the coronary blood flow, e.g. during exercise.

As was said above, the gas used as a diagnosing agent in accordance with the present invention is detectable in the expired breath from the mammal in question.

Accordingly, the gas should be present in the diagnosing agent in such an amount that the concentration thereof in the expired breath from the mammal is detectable by the desired gas monitor.

Although generally the characteristics of the gas or gas precursor can not be specified in exact figures, it should be easy for a person skilled in the art to screen candidates for gases or gas precursors without undue experimental work, as long as the gas or gas precursor is diagnostically acceptable and is of such a nature that it is detectable in the expired breath from the mammal in question. Guidance in this respect follows from the following examples of useful gases and gas precursors:
- nitrous oxide (N₂O);
- noble gases, e.g. krypton and xenon;
- lower (generally C₁-C₆) saturated or unsaturated hydrocarbons, e.g. ethane, ethene, propene and acetylene;
- sulphur hexafluoride (SF₆);
- acetone; and
- fluorinated lower hydrocarbons or hydrocarbon derivatives, e.g. volatile inhalation anesthetics such as sevoflurane (fluoromethyl-2,2,2-trifluoro-1-(trifluoromethyl)ethyl ether).
Nitrous oxide is especially preferable.

As was said above, in addition to a gas which is detectable via the expired breath from a mammal, the invention also relates to the use of a gas precursor, i.e. a substance that is the source of such a gas in connection with the diagnostic method referred to.

Both the gas and the gas precursor should be diagnostically acceptable.

The gas precursor is preferably a volatile liquid, i.e. a liquid which is readily vaporizable at a relatively low temperature, e.g. below 70 °C, or even below 40 °C or 30 °C.

The following volumes of gas or gas precursor generally gives from reasonable detection up to very good detection. The gas or gas precursor is preferably present in the diagnosing agent in a range which makes the gas well detectable but does not cause inebriation to the patient, such as for nitrous oxide a volume in the range from about 0.1 to about 2 ml, more preferably from about 0.5 to about 1 ml as measured in the gaseous state. In the case when the gas is dissolved in a diagnostically acceptable liquid, a volume between about 0.3 to about 10 ml liquid is preferable. There are no known adverse effects with short-term use of nitrous oxide, other than that the patient may fall asleep, which only may occur when much higher doses are used than those used in the present invention. For other gases, corresponding or similar volumes are applicable or can easily be determined. Particularly, more potent gases, such as sevoflurane or acetone, are preferably present in smaller volumes.

In another embodiment of the present invention, said gas or gas precursor could be dissolved in a diagnostically acceptable liquid. Dissolving the gas in a liquid reduces the risk of causing a gas embolus in the arteries, and it is also a suitable and simple way for administration of the gas. The diagnostically acceptable liquid could be Ringer's acetate, or an aqueous solution of NaCl.

In a further embodiment of the present invention, the gas could also preferably be dissolved in blood or artificial blood. More preferably, the gas could be dissolved in the mammal's own blood. In this case, there will be no dilution of the blood by another liquid, and the working myocardium will be supplied with oxygenated blood. Blood can thus be taken from the patient and mixed with an anticoagulant, such as heparin, so that the blood will not coagulate.

The administration of the diagnosing agent could be performed into any one of the left and right coronary arteries. For achieving an overall picture of how well perfused the working myocardium is, administration would, however, preferably be performed in both the left and the right coronary arteries.

More specifically, said administration of the diagnosing agent could be performed through a catheter.

As to preferable embodiments of said diagnosing agent reference is made to those preferable embodiments which have been presented in connection with the use described above. Thus, such preferable embodiments should be applicable also to the diagnosing agent per se.

Also in said method for diagnosing coronary perfusion all preferable embodiments are applicable which have been described above in connection with the use.

The gas monitoring device, or detector, to be used in connection with the present invention, could be selected among previously known gas detectors used in other connections or easily modified therefrom.

### Brief description of the drawing

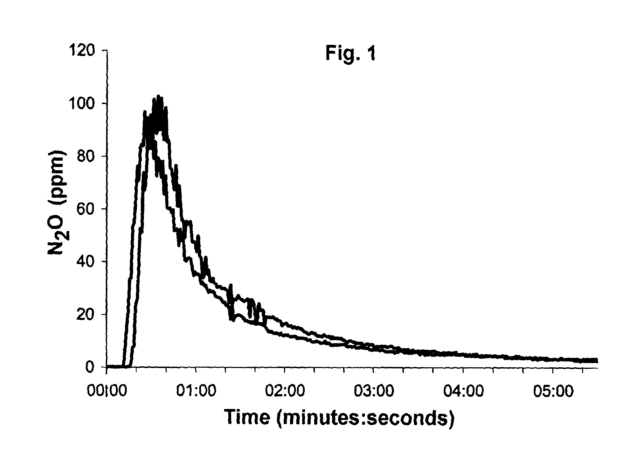

Fig. 1 shows the concentration of N₂O in exhaled air over time after injection of N₂O dissolved in Ringer's acetate to the left main coronary artery of a pig.

### Example: Detection of N₂O in exhaled air after injection of N₂O dissolved in Ringer's acetate in the left main artery

One pig was anesthetized with intravenous ketamine and pethidine, and was mechanically ventilated. A catheter was introduced to the coronary vessels via an incision in the femoral artery. After injection of an X-ray contrast medium in the catheter to check the current position within the coronary arterial system, two tests were performed by making two injections of 10 ml of Ringer's acetate, in which 1 ml of nitrous oxide had been dissolved. Nitrous oxide could be detected in the exhaled breath a few seconds later, according to figure 1. A sharp peak was detected after which the value fell exponentially, which is due to the time required to wash out nitrous oxide from the lung. This indicates that disturbances of the perfusion of blood through the myocardium can be detected by deflections of the curve showing nitrous oxide versus time.

While the invention has been illustrated by this example, it is to be understood that the invention in no aspect is limited thereto.

## Claims

1. Use of a gas, or gas precursor, which is of such a nature that it is detectable via the expired breath from a mammal, for the manufacture of a diagnostically acceptable, injectable diagnosing agent for diagnosing coronary perfusion in said mammal, including man.

2. Use according to claim 1, wherein said diagnosing is for the detection of disturbances of the coronary perfusion.

3. Use according to any one of the preceding claims, wherein said diagnosing is for the detection of stunned myocardium.

4. Use according to any one of the preceding claims, wherein said diagnosing is for the detection of myocardial infarction.

5. Use according to any one of the preceding claims, wherein said diagnosing is for the detection of angina pectoris.

6. Use according to any one of claims 1-5, wherein said gas is selected from nitrous oxide; a noble gas, preferably krypton or xenon; a lower hydrocarbon, preferably ethane, ethene, propene or acetylene; sulphur hexafluoride; and acetone.

7. Use according to claim 6, wherein said gas is nitrous oxide.

8. Use according to claim 7, wherein said nitrous oxide is present in said diagnosing agent in a volume in a range from about 0.1 to about 2 ml, as measured in the gaseous state.

9. Use according to any one of claims 1-5, wherein said gas precursor is a liquid.

10. Use according to claim 9, wherein said liquid is a fluorinated lower hydrocarbon or hydrocarbon derivative, preferably sevoflurane.

11. Use according to any one of claims 1-10, wherein said gas or gas precursor is dissolved in a diagnostically acceptable liquid.

12. Use according to claim 11, wherein said diagnostically acceptable liquid is Ringer's acetate or an aqueous solution of NaCl.

13. Use according to claim 11, wherein said diagnostically acceptable liquid is blood or artificial blood.

14. Use according to any one of the preceding claims, wherein said diagnosing agent is for administration into a coronary artery.

15. Use according to claim 14, wherein said diagnosing agent is for administration through a catheter.

16. A method for diagnosing coronary perfusion in a mammal, including man, which comprises administration to of said mammal a gas or gas precursor, which is of such a nature that it is detectable via the expired breath from said mammal, and monitoring the expired breath from said mammal by means of a gas detector to detect the gas therein.

17. A method according to claim 16, as defined in any of the claims 2-15.

18. A diagnosing agent for diagnosing coronary perfusion in a mammal, including man, which comprises a diagnostically acceptable gas or gas precursor, which is of such a nature that it is detectable via the expired breath from said mammal.

19. A diagnosing agent according to claim 18, for use as defined in any one of the claims 2-15.
